# EUROPEAN PATENT APPLICATION

(11) **EP 1 614 694 A1**
(43) Date of publication of application: **11.01.2006**
(21) Application number: 04724768.9
(22) Date of filing: 31.03.2004
(51) Int. Cl.: C07K 17/14, C12N 11/14, B01J 31/38, B01J 35/02, A61L 2/16, G01N 33/543, G01N 33/547, G01N 33/553, A61K 47/48, A61K 47/02, A61K 33/00, A61K 41/00

(54) **TITANIUM DIOXIDE COMPLEX HAVING MOLECULE DISTINGUISHABILITY**

(30) Priority: 31.03.2003 JP 2003094429; 30.09.2003 JP 2003340234
(71) Applicant: Toto Ltd., Kitakyushu-shi, Fukuoka-ken 802-8601 (JP)
(72) Inventor: SONEZAKI, Shuji, c/o Toto Ltd, Kitakyushu-shi, Fukuoka 802-8601 (JP); KANEHIRA, Koki, c/o Toto Ltd, Kitakyushu-shi, Fukuoka 802-8601 (JP); YAGI, Shinichi, c/o Toto Ltd, Kitakyushu-shi, Fukuoka 802-8601 (JP); OGAMI, Yumi, c/o Toto Ltd, Kitakyushu-shi, Fukuoka 802-8601 (JP)
(74) Representative: Gillard, Richard Edward
(86) International application number: PCT/JP2004/004638
(87) International publication number: WO 2004/087765

(57) **Abstract**

A titanium dioxide composite having a molecular recognition capacity is obtained by modifying the surface of a fine titanium dioxide particle with a hydrophilic polymer in such a manner that titanium dioxide is bonded via an ester bond to a carboxyl group of the hydrophilic polymer and immobilizing a molecule having an ability to specifically bind to a target molecule to the carboxyl residue of the hydrophilic polymer. Due to the molecule distinguishability, this titanium dioxide complex can bind specifically to an endocrine disrupting chemical, a pathogenic factor, a cancer cell and the like and decompose the same by a photocatalytic function.

## Description

### TECHNICAL FIELD

The present invention relates to a titanium dioxide composite having a molecular recognition capacity, comprising a molecule having a binding capacity specific for endocrine disrupting chemicals, etiological substances, cancer cells and the like immobilized thereon, which titanium dioxide composite can specifically bind to these substances, molecules, and cells and can degrade them, for example, upon exposure to ultraviolet light.

### BACKGROUND ART

In recent years, a material is proposed as an environment cleaning material and the material comprises a biological molecule such as DNA, having a molecular recognition capacity for endocrine disrupting chemicals, immobilized on a support to impart a selective binding property has been proposed (see, for example, Japanese Patent Laid-Open No. 81098/2001). Further, it is known that an anatase form of titanium dioxide has photocatalytic activity, and its strong oxidizing power can degrade organic matter such as microorganisms, soils, and malodorous substances. In particular, the anatase form of titanium dioxide has a high level of degradation activity even against hardly degradable materials such as endocrine disrupting chemicals and thus is expected to be effective for cleaning of environment (see, for example, Y. Ohko et al.: Environmental Science and Technology, 35, 2365-2368 (2001)). Further, at the present time, there is a technique in which the degradation efficiency of titanium dioxide is enhanced by compositing titanium dioxide with an inorganic adsorbent such as activated carbon or zeolite (see, for example, Japanese Patent Laid-Open No. 189322/1989). Regarding the surface treatment of titanium dioxide as well, a proposal has been made on the precipitation of a reduction reaction promoting catalyst metal such as palladium on the surface of a photocatalyst such as titanium dioxide to promote an oxidation/reduction reaction of the photocatalyst (see, for example, Japanese Patent Laid-Open No. 14940/1985).

For the material for selectively binding endocrine disrupting chemicals utilizing DNA or the like, however, there is no means that can reliably remove or degrade the bound endocrine disrupting chemicals and the like, and, in addition, there is a limitation on cleaning capacity due to a problem of the saturation of adsorption. Further, the technique for enhancing the capacity of titanium dioxide as a photocatalyst is also directed to neither binding nor degradation of specific substances. Accordingly, for example, selective binding to and degradation of only endocrine disrupting chemicals were impossible. Thus, in the field of environmental cleaning, any technique for selective recognition of and binding to only a target substance which is then degraded by strong oxidizing power of the photocatalyst, that is, a technique for "a combination of molecular recognition capacity with photocatalytic activity" derived from titanium dioxide, is not known in the art.

On the other hand, in recent years, a system designed so that a medicament is released in a sustained manner with the elapse of time within the body or on the surface of the body (drug delivery system: DDS) has drawn attention as a new medicament dosage form in the medical field. This system aims to maximize the efficacy of existing medicines and, at the same time, to minimize side reactions thereof. For example, as carriers for medicaments in DDS, studies have been made on nondegradable polymers and amino acid polymers (see, for example, Japanese Patent Laid-Open No. 255590/1997), liposome (see, for example, Japanese Patent Laid-Open No. 226638/2003), and protein hollow nanoparticles (see, for example, Japanese Patent Laid-Open No. 286198/2003). Target directional (targeting) DDS is an advanced system of DDS. In targeting DDS, a medicament is delivered in a necessary amount at a necessary timing to a necessary site, and, ultimately, the targeting DDS aims at a missile drug (missile therapy) which can accurately attack lesions.

In the case of the missile drug, targeting is carried out in such a manner that a ligand is carried on a DDS carrier for specific recognition of and binding to a receptor present on the surface of target cells. Ligands for target receptors in the active targeting include antigens, antibodies, peptides, glycolipids and glycoproteins. It has recently been proven that, among the above ligands, sugar chains in glycolipids and glycoproteins play an important role as information molecules in intercellular communication, for example, in proliferation and differentiation of cells, generation and morphogenesis of tissues, biophylaxis and fertilization, or canceration and its metastasis (see, for example, N. Yamazaki et al; Advanced Drug Derivery Review, 43, 225-244 (2000)).

In such DDS, an attempt has been made to apply titanium dioxide having a high level of photocatalytic degradation activity (see, N. Yamazaki et al: Advanced Drug Derivery Review, 43, 225-244 (2000), Japanese Patent Laid-Open No. 316950/2002, and R. Cai et al; Cancer Research, 52, 2346-2348 (1992)). In this method, particles of a metal such as gold supported on titanium dioxide are injected and incorporated in target cancer cells, followed by application of light such as ultraviolet light to kill the cancer cells. Titanium dioxide is known to be a material that is very stable in the air or solution and, at the same time, is nontoxic and safe within the body of an animal (i.e., in light shielded state). Further, since the activation of titanium dioxide can be controlled by on-off control of light, the application of titanium dioxide to DDS, for cancer treatment purposes is expected.

Since, however, the isoelectric point of titanium dioxide is around pH 6, titanium dioxide particles are disadvantageously agglomerated under near-neutral physiological conditions. For this reason, the administration of titanium dioxide per se directly into blood vessels or the use of the titanium dioxide particles per se as a carrier for DDS was impossible. Further, any technique for immobilizing molecules having selective binding capacity such as the above ligands on the surface of tianium dioxide is not known. Thus, at the present time, practical use of titanium dioxide as DDS is difficult. That is, also in the medical field, due to the above problems, any technique for "a combination of molecule recognition capacity with photocatalytic activity" derived from titanium dioxide, has not been developed yet.

### DISCLOSURE OF THE INVENTION

The present inventors have made extensive and intensive studies with a view to solving the above problems of the prior art and, as a result, have found that a titanium dioxide composite produced by modifying the surface of titanium dioxide fine particles with a hydrophilic polymer and then immobilizing a molecule having a binding capacity specific for a target molecule can simultaneously realize a molecular recognition capacity and a photocatalytic activity. This has led to the completion of the present invention.

Specifically, the titanium dioxide composite having a molecular recognition capacity according to the present invention comprises titanium dioxide fine particles having a surface which is modified with a hydrophilic polymer, the carboxyl groups in the hydrophilic polymer are bonded to titanium dioxide through an ester linkage, and a molecule having a binding capacity specific for a target molecule is immobilized on carboxyl residues in the hydrophilic polymer. According to this technique, a molecule having a specific binding capacity such as an antibody can be introduced into titanium dioxide particles having photocatalytic activity, and, thus, a titanium dioxide composite having a molecular recognition capacity can be produced.

The resultant titanium dioxide composite having a molecular recognition capacity can exhibit a molecular recognition capacity for endocrine disrupting chemicals, etiological substances, cancer cells and the like and, at the same time, can degrade these substances by taking advantage of its photocatalytic activity. This composite can specifically recognize and capture a target molecule in water or an aqueous solution and exhibits a very high level of degradation activity against the molecule upon exposure to ultraviolet light or the like. In particular, it should be noted that properties possessed by this composite, including that the composite can be used in aqueous solvents, can accurately recognize and capture a target molecule, and can exhibit a very high level of photocatalytic activity, are very useful for applications in the medical field, for example, degradation of harmful substances including aqueous endocrine disrupting chemicals, and destruction of specific etiological molecules and cancer cells.

### BRIEF DESCRIPTION OF THE DRAWINGS

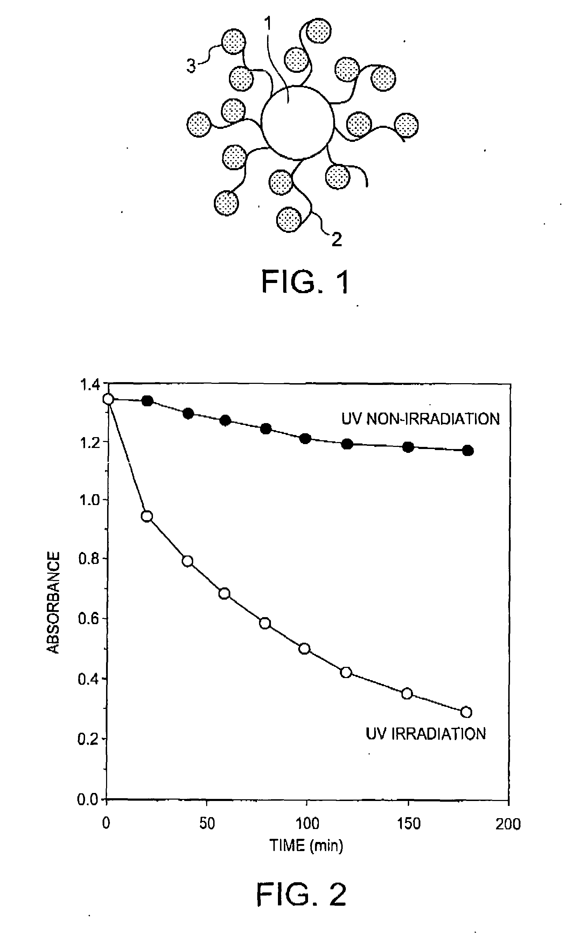

Fig. 1 is a typical diagram showing a titanium dioxide composite having a molecular recognition capacity according to the present invention;
Fig. 2 is a diagram showing degradation activity of an anti-αfetoprotein antibody-immobilized titanium dioxide composite according to the present invention against an antigen (α-fetoprotein), wherein degradation activity against the antigen is indicated in terms of a reduction in absorbance;

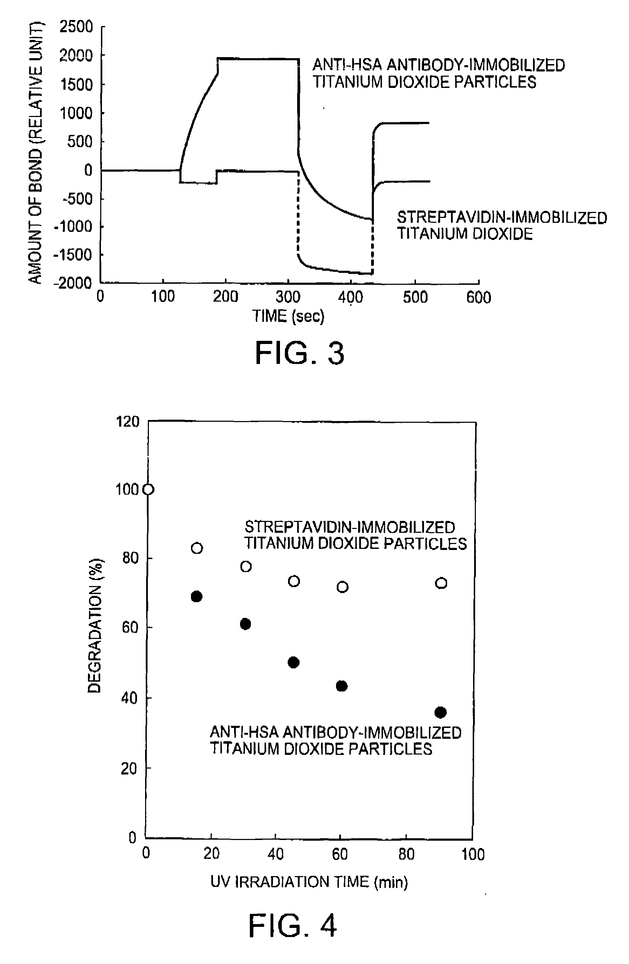

Fig. 3 is a diagram showing the results of evaluation by a surface plasmon resonance method for binding between an anti-human serum albumin antibody-immobilized titanium dioxide composite according to the present invention and an antigen (human serum albumin), wherein a streptavidin-immobilized titanium dioxide composite is used as a control;
Fig. 4 is a diagram showing the results of the determination of degradation activity of an anti-human serum albumin antibody-immobilized titanium dioxide composite according to the present invention against an antigen (human serum albumin), wherein the degradation activity against an antigen is indicated in terms of the percentage degradation (%) calculated based on a lowering in amount of bond between the antigen and the antibody by degradation (as measured by a surface plasmon resonance method) and a streptavidin-immobilized titanium dioxide composite is used as a control.

### BEST MODE FOR CARRYING OUT THE INVENTION

Embodiments of the present invention will be described in detail with reference to the accompanying drawings. Fig. 1 is a typical diagram showing a titanium dioxide composite having a molecular recognition capacity according to the present invention. Specifically, the titanium dioxide composite having a molecular recognition capacity according to the present invention is produced by dispersing titanium dioxide fine particles 1 and a hydrophilic polymer 2 containing a plurality of carboxyl groups in dimethylformamide, allowing a hydrothermal reaction to proceed at 90 to 180°C for 1 to 12 hr to bond both the materials to each other through an ester linkage, and then immobilizing a molecule 3 having a binding capacity specific for a target molecule on carboxyl residues in the hydrophilic polymer 2. Regarding the formation of an ester linkage between titanium dioxide and the hydrophilic polymer, titanium oxide on the surface of particles is hydrated by water in the reaction system to produce on its surface hydroxyl groups that are then reacted with carboxyl groups in the hydrophilic polymer. The ester linkage can be confirmed by various analytical methods. For example, in the infrared spectrophotometry, the ester linkage can be confirmed by the presence of infrared absorption around 1700 to 1800 cm⁻¹ which is an absorption band of the ester linkage. Further, the amino group in the molecule 3 having a specific binding capacity is mainly utilized for immobilization of the molecule 3. Even in the case of an amino group-free molecule, an amino group can be introduced by a proper modification method. Alternatively, a group other than the amino group, for example, a desired functional group or crosslinking reactive with the carboxyl group may also be introduced.

In the titanium dioxide fine particles 1 used in the present invention, the diameter of dispersed particles is preferably 2 to 200 nm from the viewpoints of the problem of agglomeration and the degree of freedom in type of usage, such as application in the body for cancer therapy. Both the anatase form of titanium dioxide and the rutile form of titanium dioxide are suitable as the titanium dioxide used in the present invention, because, in titanium dioxide, even in the case of different crystal systems, they are substantially identical to each other in chemical properties and, thus, modification with a water soluble polymer and immobilization of a molecule having a specific binding capacity are possible. Titanium dioxide of a desired crystal system can be selected and used depending upon applications. For example, for cancer cell destruction purposes, anatase form of titanium dioxide having a high level of photocatalytic activity can be selected.

Further, when titanium dioxide is presented on at least a part of the surface of particles, for example, even in the case of a composite composed of a magnetic material and titanium dioxide, since the properties of titanium dioxide on the particle surface resemble those in the case of titanium dioxide per se, immobilization of a molecule having a specific binding capacity through carboxyl groups is possible. Accordingly, even in the case of a composite material composed of a magnetic material and titanium dioxide, a titanium dioxide composite having a molecular recognition capacity can be produced in quite the same manner as in the case of the simple titanium dioxide particle.

The hydrophilic polymer 2 used in the present invention is preferably a water soluble polymer, because the titanium dioxide composite is contemplated to be used in the form of a dispersion in an aqueous solution. Any water soluble polymer may be used in the present invention so far as the water soluble polymer contains a plurality of carboxyl groups. Examples thereof include carboxymethyl starch, carboxymethyl dextran, carboxymethylcellulose, polycarboxylic acids, and copolymers containing carboxyl group units. More specifically, polycarboxylic acids such as polyacrylic acid and polymaleic acid, and copolymers such as acrylic acid/maleic acid copolymer and acrylic acid/sulfonic acid monomer copolymer are more preferred from the viewpoint of hydrolyzability and solubility of water soluble polymer. The modification of titanium dioxide with the above hydrophilic polymer can realize immobilization of a molecule 3 having a desired specific binding capacity on carboxyl residues in the hydrophilic polymer. Further, even after the immobilization of the molecule 3, by virtue of electrical repulsive force between the remaining carboxyl groups, the titanium dioxide composite according to the present invention can be kept in a homogeneously dispersed state over a broad pH range including near-neutral pH.

In the present invention, the molecule 3 having a specific binding capacity for imparting a molecular recognition capacity to the titanium dioxide composite is not limited to the following molecules so far as the molecule can specifically bind to the target molecule. A wide variety of such specific intermolecular bindings have been found in the living body. Among them, proteins may be mentioned as the most important molecule. In the present invention, antibodies, ligands, receptors, polyoligopeptides, and even amino acids can be immobilized as the protein. An amino group and a thiol group in the case of the immobilization of simple proteins on the titanium dioxide composite and an aldehyde group in sugar in the case of glycoproteins can be utilized as a target functional group in the immobilization. Further, a method may also be adopted in which biotin (or avidin) is introduced into carboxyl groups in titanium dioxide modified with a water soluble polymer and a protein is crosslinked with avidin (or biotin) to conduct immobilization through the utilization of interaction of biotin : avidin.

Further, in the titanium dioxide composite according to the present invention, a particular factor or ligand may be present on the particle surface. Accordingly, for example, for cells expressing a specific receptor such as cancer cells, this composite can be introduced into the cells through specific binding of ligand : receptor. These factors and ligands include proliferation and growth factors and formation factors, such as epidermal growth factors (EGFs), transforming growth factors, platelet-derived growth factors, osteogenetic factors, and nerve growth factors, and, in addition, hormones and ligands, such as interferons, interleukins, colony stimulating factors, tumor necrosis factors, erithropoietin, Fas antigens, and activins. These proteins can also be immobilized in the same manner as described above. Specifically, a missile drug can be constructed which can realize targeting to specific cells specifically expressing receptors corresponding to them.

In recent years, attention has been drawn to a nucleic acid aptamer which can specifically bind to a specific protein. This aptamer also can be utilized as the molecule 3 having a specific binding capacity for imparting a molecular recognition capacity according to the present invention. The nucleic acid can be immobilized on a modified titanium dioxide in the same manner as described above by, in the amplification of DNA by a polymerase chain reaction (PCR), synthesizing a modified DNA using an amination primer, a biotinylation primer, or a thiolation primer. For example, when aminated DNA is used in the immobilization, a method may be used in which an ester such as N-hydroxysuccinimide (NHS) is previously introduced into carboxyl groups in the modified titanium dioxide and the aminated DNA can be covalently bonded to the modified titanium dioxide by a nucleophilic displacement reaction. Also when the thiolated DNA is used, likewise, the thiolated DNA can be immobilized on the modified titanium dioxide by reacting carboxyl groups with NHS and then allowing 2-(2-pyridinyldithio)ethaneamine to act thereon.

When an aldehyde group in the molecule to be immobilized is used, a method may be used in which, after NHS is reacted with carboxyl groups, the molecule to be immobilized is bonded to the modified titanium dioxide using hydrazine followed by a reduction with sodium cyanoboride. Alternatively, a method may be used in which carboxyl groups are biotinylated by using biotin hydrazide or aminated biotin and the avidinated molecule can then be easily immobilized on the modified titanium dioxide. Thus, a wide variety of molecules 3 having a specific binding capacity can be easily immobilized on carboxyl residues introduced onto the modified titanium dioxide by properly selecting reagents, and modification and crosslinking methods.

As described above, in addition to proteins and nucleic acid or saccharides, lipids and various physiologically active substances and the like can be suitably used as the molecule (3) having a specific binding capacity so far as they contain a functional group which can bind to carboxyl residues introduced onto the modified titanium dioxide and the bonding method is known.

On the other hand, the titanium dioxide composite should be homogeneously dispersed in a neutral aqueous solvent from the viewpoint of physiological conditions within the living body, when the application of the titanium dioxide composite having a molecular recognition capacity according to the present invention in aqueous harmful substance treatment or in medicaments or medical procedures is contemplated. As described above, the titanium dioxide composite having a molecular recognition capacity according to the present invention contains the remaining carboxyl residues, in the aqueous solvent. Therefore, the repulsive force derived from the negative charge in the carboxyl group acts on between composites. Thus, the composite can be kept in a homogeneously dispersed state without agglomeration even in an aqueous solution over a wide pH range, pH 3 to 13. Accordingly, a homogenous and stable dispersion liquid prepared by dispersing the titanium dioxide composite having a molecular recognition capacity according to the present invention in water, various pH buffer solutions, transfusions, or physiological saline can be provided. Further, for example, ointment and spray preparations containing this dispersion liquid can also be produced. The above property is particularly useful for the application of titanium dioxide in in-vivo and in-vitro DDS. Specifically, the dispersion liquid of the titanium dioxide composite having a molecular recognition capacity according to the present invention is not agglomerated even under near-neutral physiological conditions and thus can be injected directly into an affected tissue or intravenously injected for targeting. Further, an ointment or spray preparation containing this dispersion liquid can be applied directly to affected parts such as skin followed by phototherapy using sunlight, an ultraviolet light lamp or the like.

Further, the titanium dioxide composite having a molecular recognition capacity according to the present invention can of course be utilized solely as DDS, or alternatively may be included as one form of DDS in other carrier. In this case, the carrier is not particularly limited, but for example, liposomes, virus particles, and hollow nanoparticles are preferably used.

Any special light source device is not required for exciting and activating the titanium dioxide composite having a molecular recognition capacity according to the present invention, but the wavelength is preferably not more than 400 nm from the viewpoint of the bandgap of titanium dioxide. In external applications in skin and the like, sunlight, conventional ultraviolet lamps, and black light are suitable. In the case of the affected part within the body, ultraviolet light may be applied by mounting an ultraviolet fiber on an endoscope. Further, when phototherapy in which ultraviolet light particularly at around 280 nm is locally applied to the affected part to destruct the affected region is contemplated, the titanium dioxide composite having a molecular recognition capacity according to the present invention may be applied as an action enhancing agent.

The following Examples further illustrate the present invention but do not limit the present invention.

### Example 1

### Introduction of polyacrylic acid into titanium dioxide particles

Titanium tetraisopropoxide (3.6 g) and 3,6 g of isopropanol were mixed together, and the mixture was added dropwise to 60 ml of ultrapure water under ice cooling for hydrolysis. After the completion of the dropwise addition, the reaction solution was stirred at room temperature for 30 min. After the stirring, 1 ml of 12 N nitric acid was added dropwise thereto, and the mixture was stirred at 80°C for 8 hr for peptization. After the completion of the peptization, the reaction solution was filtered through a 0.45-µm filter, followed by solution exchange through a desalination column (PD10; Amersham Biosciences K.K.) to prepare an anatase-form titanium dioxide sol having a solid content of 1 %. This dispersion liquid was placed in a 100 ml-volume vial bottle and was ultrasonicated at 200 Hz for 30 min. The average diameter of the dispersed particles before the ultrasonication and the average diameter of the dispersed particles after the ultrasonication were 36.4 nm and 20.2 nm, respectively. After the completion of the ultrasonication, the solution was concentrated to prepare a titanium dioxide sol (anatase form) having a solid content of 20%.

The titanium dioxide sol (0.75 ml) thus obtained was dispersed in 20 ml of dimethylformamide (DMF). Polyacrylic acid (average molecular weight: 5000, Wako Pure Chemical Industries, Ltd.) (0.2 g) dissolved in 10 ml of DMF was added to the dispersion liquid, followed by stirring for mixing. The solution was transferred to a hydrothermal reaction vessel, and hydrothermal synthesis was allowed to proceed at 180°C for 6 hr. After the completion of the reaction, the reaction vessel was cooled to 50°C or below. The solution was taken out of the reaction vessel, 80 ml of water was then added to the solution, followed by stirring for mixing. DMF and water were removed by an evaporator. Thereafter, 20 ml of water was again added to the residue to prepare an aqueous polyacrylic acid-modified titanium dioxide solution. 2 N hydrochloric acid (1 ml) was added to the aqueous solution to precipitate titanium dioxide particles, and the mixture was centrifuged. The supernatant was then removed to separate polyacrylic acid remaining unreacted. Water was again added for washing, the mixture was centrifuged, and water was then removed. A 50 mM phosphate buffer solution (pH 7.0) (10 ml) was added thereto, and the mixture was then ultrasonicated at 200 kHz for 30 min to disperse the titanium dioxide particles. After the completion of the ultrasonication, the dispersion liquid was filtered through a 0.45-µm filter to prepare a polyacrylic acid-modified titanium dioxide sol having a solid content of 1.5%. The diameter of the dispersed polyacrylic acid-modified titanium dioxide fine particles (anatase form) thus obtained was measured and was found to be 45.5 nm.

### Example 2

### Immobilization of anti-AFP antibody molecule on polyacrylic acid-modified titanium dioxide fine particles

The polyacrylic acid-modified titanium dioxide sol (anatase form) (1 ml) prepared in Example 1 was subjected to solution exchange through a desalination column PD10 to prepare 3 ml of a polyacrylic acid-modified titanium dioxide sol dispersed in water. A mixed liquid (0.1 ml) composed of 200 mM 1-ethyl-3-(3-dimethylaminopropyl)carbodiimide and 50 mM N-hydroxysuccinimide (NHS) was added to 1.5 ml of this solution. The mixture was stirred for 10 min to activate the carboxyl groups. After the stirring, the reaction solution was subjected to solution exchange through PD10 equilibrated with a 10 mM acetate buffer solution (pH 5.0) to give 3 ml of a carboxyl-activated polyacrylic acid-modified titanium dioxide sol dispersed in a 10 mM acetate buffer solution (pH 5.0). An anti-α-fetoprotein (anti-AFP) polyclonal antibody (goat IgG, SC-8108; Cosmo-Bio Co., Ltd.) prepared using the same buffer solution was added to the sol to a concentration of 0.05 mg/ml. The mixture was stirred at room temperature for 15 min, and an aqueous ethanolamine hydrochloride solution (pH 8.5) was then added to the mixture to a concentration of 0.5 M. After stirring for 10 min, 1 ml of 2 N hydrochloric acid was added to precipitate titanium dioxide particles, followed by centrifugation. The supernatant was then removed. Water was again added for washing, the mixture was centrifuged, and water was then removed. A 50 mM phosphate buffer solution (pH 7.0) (2.5 ml) was added, and the mixture was then ultrasonicated at 200 Hz for 30 min to disperse titanium dioxide particles. After the ultrasonication, the mixture was filtered through a 0.45-µm filter to give an anti-AFP antibody-immobilized titanium dioxide composite sol having a solid content of 0.3%. The diameter of dispersed particles of the anti-AFP antibody-immobilized titanium dioxide composite (anatase form) thus obtained was measured and found to be 52.8 nm.

### Example 3

### immobilization of anti-HSA antibody molecule on polyacrylic acid-modified titanium dioxide fine particles

The polyacrylic acid-modified titanium dioxide sol (anatase form) (1 ml) prepared in Example 1 was subjected to solution exchange through a desalination column PD10 to prepare 3 ml of a polyacrylic acid-modified titanium dioxide sol dispersed in water. A mixed liquid (0.1 ml) composed of 200 mM 1-ethyl-3-(3-dimethylaminopropyl)carbodiimide and 50 mM N-hydroxysuccinimide (NHS) was added to 1.5 ml of this solution. The mixture was stirred for 10 min to activate the carboxyl groups. After the stirring, the reaction solution was subjected to solution exchange through PD10 equilibrated with a 10 mM acetate buffer solution (pH 5.0) to give 3 ml of a carboxyl-activated polyacrylic acid-modified titanium dioxide sol dispersed in a 10 mM acetate buffer solution (pH 5.0). An anti-human serum albumin (anti-HSA) monoclonal antibody (mouse IgG, MSU-304; Cosmo-Bio Co., Ltd.) prepared using the same buffer solution was added to the sol to a concentration of 0.05 mg/ml. The mixture was stirred at room temperature for 15 min, and an aqueous ethanolamine hydrochloride solution (pH 8.5) was then added to the mixture to a concentration of 0.5 M. After stirring for 10 min, 2.5 M NaCl and 20%(w/v) polyethylene-glycol were added in equal amounts to precipitate titanium dioxide particles, followed by centrifugation. The supernatant was then removed. Water was again added for washing, the mixture was centrifuged, and water was then removed. A PBS buffer solution (pH 7.0: containing 100 mM NaCl, NIPPON GENE CO., LTD) (2.5 ml) was added to disperse titanium dioxide particles. The dispersion was filtered through a 0.45-µm filter to give an anti-HSA antibody-immobilized titanium dioxide composite sol having a solid content of 0.3%. The diameter of dispersed particles of the anti-HSA antibody-immobilized titanium dioxide composite (anatase form) thus obtained was measured and found to be 52.8 nm.

### Example 4

### Immobilization of streptavidin molecule on polyacrylic acid-modified titanium dioxide fine particles

The polyacrylic acid-modified titanium dioxide sol (anatase form) (1 ml) prepared in Example 1 was subjected to solution exchange through a desalination column PD10 to prepare 3 ml of a polyacrylic acid-modified titanium dioxide sol dispersed in water. A mixed liquid (0.1 ml) composed of 200 mM 1-ethyl-3-(3-dimethylaminopropyl)carbodiimide and 50 mM N-hydroxysuccinimide (NHS) was added to 1.5 ml of this solution. The mixture was stirred for 10 min to activate the carboxyl groups. After the stirring, the reaction solution was subjected to solution exchange through PD10 equilibrated with a 10 mM acetate buffer solution (pH 5.0) to give 3 ml of a carboxyl-activated polyacrylic acid-modified titanium dioxide sol dispersed in a 10 mM acetate buffer solution (pH 5.0). Streptavidin (Pierce Biotechnology Inc., code: 21126) was added to the sol to a concentration of 0.05 mg/ml. The mixture was stirred at room temperature for 15 min, and an aqueous ethanolamine hydrochloride solution (pH 8.5) was then added to the mixture to a concentration of 0.5 M. After stirring for 10 min, 2.5 M NaCl and 20%(w/v) polyethylene-glycol were added in equal amounts to precipitate titanium dioxide particles, followed by centrifugation. The supernatant was then removed. Water was again added for washing, the mixture was centrifuged, and water was then removed. A PBS buffer solution (pH 7.0, NIPPON GENE CO., LTD) (2.5 ml) was added to disperse titanium dioxide particles. The dispersion was filtered through a 0.45-µm filter to give a streptavidin-immobilized titanium dioxide composite sol having a solid content of 0.3%. The diameter of dispersed particles of the streptavidin-immobilized titanium dioxide composite (anatase form) thus obtained was measured and found to be 50.5 nm.

### Example 5

### Synthesis of polyacrylic acid-modified magnetic material composite titanium dioxide fine particles

Polyoxyethylene(15) cetyl ether (C-15; NIHON SURFACTANT KOGYO K.K.) (45.16 g) was dissolved within a separable flask. The air in the separable flask was replaced by nitrogen for 5 min. A cyclohexene solution (Wako Pure Chemical Industries, Ltd.) (75 ml) was then added to the solution, and 3.6 ml of a 0.67 M aqueous FeCl₂ (Wako Pure Chemical Industries, Ltd.) solution was added thereto. A 30% aqueous ammonia solution (5.4 ml) was added to the mixture with stirring at 250 rpm, and a reaction was allowed to proceed for one hr. Thereafter, 0.4 ml of a 50 mM aqueous tetraethyl orthosilicate solution (Wako Pure Chemical Industries, Ltd.) was added dropwise thereto, and a reaction was allowed to proceed for one hr. Thereafter, titanium tetraisopropoxide (Wako Pure Chemical Industries, Ltd.) was added to a final concentration of 0.005 M. A 50% (w/v) aqueous ethanol solution (10 ml) was added in 1 ml portions at intervals of 10 min. The reaction solution was centrifuged, and the precipitate was fired at 350°C for 2 hr. After the completion of the firing, the fired product was dispersed in a 10 mM aqueous nitric acid solution, and the dispersion liquid was ultrasonicated, followed by filtration through a 0.1-µm filter. The magnetic material/titanium dioxide composite sol (0.75 ml) thus obtained was dispersed in 20 ml of dimethylformamide (DMF), and a solution of 0.3 g of polyacrylic acid (average molecular weight: 5000, Wako Pure Chemical Industries, Ltd.) dissolved in 10 ml of DMF was added to the dispersion liquid, followed by stirring for mixing. The solution was transferred to a hydrothermal reaction vessel (HU-50, SAN-Al Science Co. Ltd.), and synthesis was allowed to proceed at 180°C for 6 hr. After the completion of the reaction, the reaction vessel was cooled to 50°C or below. The solution was taken out of the reaction vessel and was placed in a separatory funnel, 10 ml of water was then added thereto, followed by stirring for mixing. Next, 40 ml of chloroform was added to and mixed with the mixture while stirring, and the lower layer was then removed to recover the upper layer. This step was repeated twice to remove DMF. To 10 ml of this solution were added 10 ml of 1.5 M NaCl and 20% (w/v) polyethylene-glycol 6000 (Wako Pure Chemical Industries, Ltd.). The mixture was centrifuged, and the supernatant was then removed. Water (2.5 ml) was added to the precipitate, and the mixture was subjected to gel filtration through a Sephadex G-25 column to prepare a dispersion liquid of polyacrylic acid-modified magnetic material composite titanium dioxide fine particles (anatase form).

### Example 6

### Immobilization of anti-HSA antibody molecule on polyacrylic acid-modified magnetic material composite titanium dioxide fine particles

A mixed liquid (0.1 ml) composed of 200 mM 1-ethyl-3-(3-dimethylaminopropyl)carbodiimide and 50 mM N-hydroxysuccinimide (NHS) was added to 1.5 ml of the dispersion liquid of polyacrylic acid-modified magnetic material composite titanium dioxide fine particles prepared in Example 5. The mixture was stirred for 10 min to activate the carboxyl groups. After the stirring, the reaction solution was subjected to solution exchange through PD10 equilibrated with a 10 mM acetate buffer solution (pH 5.0) to give 3 ml of a carboxyl-activated polyacrylic acid-modified magnetic material composite titanium dioxide sol dispersed in a 10 mM acetate buffer solution (pH 5.0). An anti-human serum albumin (anti-HSA) monoclonal antibody (mouse IgG, MSU-304; Cosmo-Bio Co., Ltd.) prepared using the same buffer solution was added to the sol to a concentration of 0.05 mg/ml. The mixture was stirred at room temperature for 15 min, and an aqueous ethanolamine hydrochloride solution (pH 8.5) was then added to the mixture to a concentration of 0.5 M. After stirring for 10 min, 2.5 M NaCl and 20%(w/v) polyethylene-glycol were added in equal amounts to precipitate magnetic material composite titanium dioxide particles, followed by centrifugation. The supernatant was then removed. Water was again added for washing, the mixture was centrifuged, and water was then removed. PBS (NIPPON GENE CO., LTD) (2.5 ml) was added to disperse magnetic material composite titanium dioxide particles. The dispersion was filtered through a 0.45-µm filter to give an anti-HSA antibody-immobilized magnetic material composite titanium dioxide composite sol having a solid content of 0.3%. The diameter of dispersed particles of the anti-HSA antibody-immobilized magnetic material composite titanium dioxide composite (anatase form) thus obtained was measured and found to be 105 nm.

### Example 7

### Introduction of acrylic acid/sulfonic acid copolymer into titanium dioxide particles

The titanium dioxide sol (anatase form) having a solid content of 20% (0.75 ml) produced in the process of Example 1 was dispersed in 20 ml of dimethylformamide (DMF). An acrylic acid/sulfonic acid monomer copolymer (manufactured by Nippon Shokubai Kagaku Kogyo Co., Ltd.; average molecular weight: 5000; a preparation obtained by replacing with proton followed by lyophilization) (0.3 g) dissolved in 10 ml of DMF was added to the dispersion liquid, followed by mixing with stirring. The solution was transferred to a hydrothermal reaction vessel (HU-50, SAN-Al Science Co. Ltd.), and synthesis was allowed to proceed at 150°C for 5 hr. After the completion of the reaction, the reaction vessel was cooled to room temperature. Isopropanol (Wako Pure Chemical Industries, Ltd.) in an amount of twice the amount of the reaction solution was added to the reaction solution. The mixture was left to stand at room temperature for 30 min or longer, followed by centrifugation under conditions of 4000 x g and 20 min to collect precipitates. The collected precipitates were washed with 70% ethanol, and 2.5 ml of water was then added to prepare an acrylic acid/sulfonic acid copolymer-modified titanium dioxide sol (anatase form).

### Example 8

### Immobilizatin of anti-DR4 antibody molecule on acrylic acid/sulfonic acid copolymer-modified titanium dioxide fine particles

A mixed liquid (0.1 ml) composed of 200 mM 1-ethyl-3-(3-dimethylaminopropyl)carbodiimide and 50 mM N-hydroxysuccinimide (NHS) was added to 1.5 ml of the acrylic acid/sulfonic acid copolymer-modified titanium dioxide sol prepared in Example 7. The mixture was stirred for 10 min to activate the carboxyl groups. After the stirring, the reaction solution was subjected to solution exchange through PD10 equilibrated with a 10 mM acetate buffer solution (pH 5.0) to give 3 ml of a carboxyl-activated acrylic acid/sulfonic acid copolymer-modified titanium dioxide sol dispersed in a 10 mM acetate buffer solution (pH 5.0). An anti-DR4 monoclonal antibody (Anti-TRAIL Receptor 1, mouse, code: SA-225, FUNAKOSHI CO. LTD.) was added to the sol to a concentration of 0.05 mg/ml, The mixture was stirred at room temperature for one min, and an aqueous ethanolamine hydrochloride solution (pH 8.5) was then added to the mixture to a concentration of 0.5 M. After stirring for 10 min at room temperature, 2.5 M NaCl and 20% (w/v) polyethylene-glycol were added in equal amounts to precipitate titanium dioxide particles, followed by centrifugation. The supernatant was then removed. Water was added for washing, and the mixture was then centrifuged to collect precipitates. A PBS buffer solution (pH 7.0, NIPPON GENE CO., LTD.) (2.5 ml) was added to disperse titanium dioxide particles. The dispersion was filtered through a 0.45-µm filter to give an anti-DR4 antibody-immobilized titanium dioxide composite sol (anatase form) having a solid content of 0.3%.

### Example 9

### Degradation of antigen AFP by anti-AFP antibody-immobilized titanium dioxide composite

α-Fetoprotein (AFP, Cosmo-Bio Co., Ltd.) was diluted with a 50 mM PBS buffer solution (pH 7.0, NIPPON GENE CO., LTD.) to a concentration of 1 µg/ml, and the anti-AFP antibody-immobilized titanium dioxide composite prepared in Example 2 was added thereto to a solid content of 0.01%. Subsequently, the mixture was left to stand at 37°C for 3 hr to form an agglomerate produced as a result of an antigen-antibody reaction. The formation of an agglomerate by AFP and the anti-AFP antibody-immobilized titanium dioxide composite demonstrates that the anti-AFP antibody-immobilized titanium dioxide composite specifically recognized and was bound to AFP. Ultraviolet light with a wavelength of 340 nm was applied at 1 mW/cm² to the agglomerate with stirring, and wavelength absorption at 600 nm (turbidity of the agglomerate) was measured with a spectrophotometer. The results are shown in Fig. 2. Only when ultraviolet light (UV) was applied, a reduction in absorbance derived from a lowering in aggolomerate concentration was observed, demonstrating that the antigen AFP was degraded by photocatalytic action of the anti-AFP antibody-immobilized titanium dioxide composite.

### Example 10

### Confirmation of antigen-antibody reaction by anti-HSA antibody-immobilized titanium dioxide composite

Human serum albumin (HSA, Cosmo-Bio Co., Ltd.) was diluted with a 50 mM PBS buffer solution (pH 7.0, NIPPON GENE CO., LTD.) to a concentration of 250 µg/ml. Separately, a sensor chip C1 (Biacore K.K.) in a surface plasmon resonance sensor was activated by a mixed liquid composed of 400 mM 1-ethyl-3-(3-dimethylaminopropyl)carbodiimide and 100 mM N-hydroxysuccinimide (NHS). This sensor chip was mounted on a surface plasmon resonance measuring apparatus: BIACORE 1000 (Biacore K.K.). The HSA solution prepared above was passed through the apparatus at a flow rate of 10 µl/min, and blocking of active groups was then carried out with 0.1 M ethanolamine to prepare an HSA-immboilized sensor chip. The 0.01% anti-HSA antibody-immobilized titanium dioxide composite sol prepared in Example 3 and the 0.01% streptavidin-immobilized titanium dioxide composite sol prepared in Example 4 were supplied to the HSA-immobilized sensor chip to confirm an antigen-antibody reaction. The results are shown in Fig. 3. The anti-HSA antibody-immobilized titanium dioxide composite was reacted with and bonded to the HSA-immobilized sensor chip, whereas the streptavidin-immobilized titanium dioxide composite was not reacted with and not bonded to the HSA-immobilized sensor chip, confirming that the anti-HSA monoclonal antibody immobilized on the hydrophilic polymer on titanium dioxide could surely maintain activity as the antibody after the immobilization.

### Example 11

### Degradation of antigen HSA by anti-HSA antibody-immobilized titanium dioxide composite

HSA was diluted with a PBS buffer solution (pH 7.0, NIPPON GENE CO., LTD.) to a concentration of 20 ng/ml, and the anti-HSA antibody-immobilized titanium dioxide composite prepared in Example 3 was added thereto to a solid content of 0.01%. Subsequently, the mixture was allowed to stand at room temperature for 30 min and was then exposed to ultraviolet light at 1 mW/cm² with a wavelength of 340 nm. In this case, sampling was carried out every 15 min over a period of 90 min. The same treatment was carried out for the streptavidin-immobilized titanium dioxide composite prepared in Example 4. Separately, an anti-HSA polyclonal antibody (rabbit)-immobilized sensor chip for surface plasmon resonance measurement was prepared in the same manner as in Example 8. In the same manner as in Example 8, BIACORE 1000 was used, and 20 µl of a sample for the elapse of each time period was supplied to the anti-HSA antibody-immobilized sensor chip. Subsequently, 10 µl of a 50 µg/ml anti-HSA polyclonal antibody (rabbit) as a secondary antibody was supplied for sandwich assay, and, 10 sec after the supply of the antibody, RU value (corresponding to the amount of bond) was measured. The percentage HSA degradation calculated based on the relative value determined by taking RU value for UV unirradiation to be 100% is shown in Fig. 4. The results shown in Fig. 4 show that, as compared with the streptavidin-immobilized titanium dioxide composite, the anti-HSA antibody-immobilized titanium dioxide composite has much higher HSA degradation rate.

### INDUSTRIAL APPLICABILITY

The present invention provides a titanium dioxide composite having a molecular recognition capacity, which specifically binds to endocrine disrupting chemicals, etiological substances, cancer cells and the like and can degrade them by taking advantage of its photocatalytic activity.

## Claims

1. A titanium dioxide composite having a molecular recognition capacity, comprising titanium dioxide having a surface which is modified with a hydrophilic polymer having carboxyl groups, the carboxyl groups in the hydrophilic polymer being bonded to titanium dioxide through an ester linkage, a molecule having a binding capacity specific for a target molecule being immobilized on the carboxyl groups in the hydrophilic polymer.

2. The titanium dioxide composite having a molecular recognition capacity according to claim 1, wherein said titanium dioxide is an anatase or rutile form of titanium dioxide.

3. The titanium dioxide composite having a molecular recognition capacity according to claim 1 or 2, wherein said titanium dioxide has a particle diameter of 2 to 200 nm.

4. The titanium dioxide composite having a molecular recognition capacity according to any one of claims 1 to 3, wherein said titanium dioxide is a composite titanium dioxide comprising titanium dioxide and a magnetic material.

5. The titanium dioxide composite having a molecular recognition capacity according to any one of claims 1 to 4, wherein said hydrophilic polymer is a water soluble polymer.

6. The titanium dioxide composite having a molecular recognition capacity according to claim 5, wherein said water soluble polymer contains a polycarboxylic acid.

7. The titanium dioxide composite having a molecular recognition capacity according to claim 5, wherein said water soluble polymer comprises a copolymer having a plurality of carboxyl group units in its molecule.

8. The titanium dioxide composite having a molecular recognition capacity according to any one of claims 1 to 7, wherein the molecule having a binding capacity specific for a target molecule is an amino acid, a peptide, a simple protein, a complex protein, or an antibody.

9. The titanium dioxide composite having a molecular recognition capacity according to any one of claims 1 to 7, wherein the molecule having a binding capacity specific for a target molecule is a nucleoside, a nucleotide, a nucleic acid, or a peptide nucleic acid.

10. The titanium dioxide composite having a molecular recognition capacity according to any one of claims 1 to 7, wherein the molecule having a binding capacity specific for a target molecule is a monosaccharide, a sugar chain, a polysaccharide, and a complex carbohydrate.

11. The titanium dioxide composite having a molecular recognition capacity according to any one of claims 1 to 7, wherein the molecule having a binding capacity specific for a target molecule is a fatty acid, a fatty acid derivative, a simple lipid, and a complex lipid.

12. The titanium dioxide composite having a molecular recognition capacity according to any one of claims 1 to 7, wherein the molecule having a binding capacity specific for a target molecule is a physiologically active substance.

13. A dispersion liquid of a titanium dioxide composite having a molecular recognition capacity, wherein comprising the titanium dioxide composite having a molecular recognition capacity according to any one of claims 8 to 12, contained in an aqueous solution of which the introduction into a living body is acceptable.

14. The dispersion liquid of a titanium dioxide composite having a molecular recognition capacity according to claim 13, wherein the aqueous solution is a pH buffer solution.

15. The dispersion liquid of a titanium dioxide composite having a molecular recognition capacity according to claim 13, wherein the aqueous solution is physiological saline.

16. The dispersion liquid of a titanium dioxide composite having a molecular recognition capacity according to any one of claims 13 to 15, wherein the titanium dioxide composite having a molecular recognition capacity is included in an inclusion material of which the introduction into a living body is acceptable.

17. The dispersion liquid of a titanium dioxide composite having a molecular recognition capacity according to claim 16, wherein said inclusion material is any of a liposome, a virus particle, and a hollow nanoparticle.
